# FASCICULE DE BREVET EUROPEEN

(11) **EP 1 102 581 B1**
(45) Date de publication et mention de la délivrance du brevet: **22.05.2002**
(21) Numéro de dépôt: 99934821.2
(22) Date de dépôt: 30.07.1999
(51) Int. Cl.: A61K 9/48, A61K 9/107

(54) **COMPOSITION A LIBERATION PROLONGEE DE PRINCIPE ACTIF APTE A FORMER UNE MICRO-EMULSION**
ZUR BILDUNG EINER MIKROEMULSION FÄHIGEN ZUSAMMENSETZUNG MIT VERLÄNGERTER FREISETZUNG DES WIRKSTOFFS
A MICROEMULSION FORMING COMPOSITION FOR THE SUSTAINED RELEASE OF AN ACTIVE AGENT

(30) Priorité: 07.08.1998 FR 9810313
(43) Date de publication de la demande: 30.05.2001
(73) Titulaire: GATTEFOSSE HOLDING, 69800 St Priest (FR)
(72) Inventeur: BARTHELEMY, Philippe, F-69780 Mions (FR); BENAMEUR, Hassan, F-69740 Genas-Azieu (FR)
(74) Mandataire: Vuillermoz, Bruno
(86) Numéro de dépôt international: FR9901889
(87) Numéro de publication internationale: WO0007573

(56) Documents cités:
- EP-A- 0 222 614
- EP-A- 0 806 202
- WO-A-95/08983
- WO-A-96/21439
- WO-A-99/12528
- US-A- 5 206 219

## Description

L'invention concerne une composition à libération prolongée de principe actif administrable notamment par voie orale, à usage pharmaceutique ou cosmétique, apte à former une micro-émulsion avec une phase hydrophile externe, par exemple le liquide physiologique ou l'eau, tout en libérant progressivement l'actif qu'elle contient in situ. Elle se rapporte également au procédé de fabrication de ladite composition.

Comme on le sait, une micro-émulsion est une solution homogène, fluide et stable, constituée de quatre constituants majeurs respectivement une phase hydrophile, une phase lipophile, au moins un agent tensio-actif (TA) et au moins un agent cotensio-actif. Les micro-émulsions se distinguent des émulsions et des solutions micellaires notamment de par la taille des gouttelettes dont elles sont constituées. En effet, la taille des gouttelettes d'une micro-émulsion est comprise entre 10 et 200 nanomètres (nm) alors qu'elle est inférieure à 10 nm pour une solution micellaire et supérieure à 200 nm pour une émulsion. Par ailleurs, à la différence des émulsions, qui sont instables, les micro-émulsions, lesquelles comprennent nécessairement un agent cotensio-actif, sont stables. De plus, on caractérise une micro-émulsion de par sa transparence plus ou moins accentuée due à la proportion de lumière réfléchie transmise par un faisceau lumineux, l'intensité du faisceau lumineux ayant traversé étant inférieure à celle du faisceau incident. La lumière réfléchie étant plus riche en radiations bleues et violettes, les micro-émulsion finement dispersées prennent un aspect bleuté. C'est l'effet dit TYNDALL décrit notamment dans l'ouvrage « Emulsions, micro-émulsions, émulsions multiples » de Jean Poré.

Le Demandeur a décrit dans le document EP-A-0 670 715 une composition formée d'un système vecteur d'actif auto-micro-émulsionable connu sous l'expression britannique SMEDDS®, marque déposée par le Demandeur signifiant Self Micro Emulsifying Drug Delivery System. Ces systèmes sont longuement décrits dans le document précité et comprennent pour l'essentiel :
- un actif,
- une phase lipophile constituée d'un mélange de mono-, di- et triglycérides et des acides gras en C₈-C₁₈ et de mono- et diesters de polyéthylène glycol présentant une balance hydrophile/lipophile (HLB) inférieure à 16 ;
- un agent tensio-actif (TA) à base de glycérides présentant une HLB inférieure à 16 choisi dans le groupe comprenant les glycérides polyglycolysés saturés en C₈-C₁₀ et les esters oléïques du polyglycérol ;
- un agent cotensio-actif (CoTA) choisi dans le groupe comprenant les esters lauriques du propylène glycol, les esters oléïques du polyglycérol, l'éthyl diglycol et le polyéthylène glycol ;
- le rapport TA-CoTA étant compris entre 0,5 et 6.

Certains produits commercialisés par le Demandeur, constitués d'acides gras saturés et/ou insaturés et d'esters de ces acides gras, peuvent être utilisés en tant que phase lipophile, agent tensioactif et agent co-tensioactif, telle que par exemple l'association respectivement de GELUCIRE 44/14, LABRAFAC CM10 et LAUROGLYCOL décrits dans les exemples 1 et 2 du document suscité.

Les SMEDDS® peuvent se présenter à température ambiante sous forme solide ou liquide, en fonction de la nature même des corps gras qui les composent. Ainsi, si au moins un des corps gras constituant du SMEDDS® présente un point de fusion supérieur à la température ambiante (environ 25°C) alors le SMEDDS® sera semi-solide à température ambiante. Au contraire, si au moins un corps gras constituant du SMEDDS® présente un point de fusion inférieur à environ 25°C, alors le SMEDDS® est liquide à température ambiante.

En conséquence, les SMEDDS® peuvent être incorporés dans des gélules à l'état liquide, éventuellement à chaud, puis, en fonction de la nature de leurs constituants, restent liquides ou deviennent semi-solides à température ambiante.

De par la formation de la micro-émulsion in situ, les SMEDDS® permettent de solubiliser le principe actif et par conséquent d'augmenter la biodisponibilité du ou des actifs micro-émulsionnés qu'ils véhiculent. Cependant, la formation de la micro-émulsion confère à la composition des caractéristiques de libération immédiate d'actif micro-émulsionné.

Le document WO 96/21439 décrit une composition pharmaceutique pour le traitement de l'hyperlipidémie comprenant un mélange de fénofibrate, de glycérides polyglycosylés (notamment GELUCIRE® 44/14) et de dérivés de cellulose ou de polyoxamères. Dans ce document, les dérivés de cellulose sont utilisés en tant qu'agents stabilisants, permettant d'éviter la formation de cristaux de fénofibrate durant le refroidissement de la capsule dure.

Le document EP-A-0 222 614 décrit une association de GELUCIRE® 46/07 commercialisé par GATTEFOSSE et d'un agent hydrophile. Le GELUCIRE® 46/07 ne peut être considéré comme un SMEDDS®, dans la mesure où il ne contient notamment pas de phase cotensio-active, telle que esters de propylène glycol, ester oléique de polyglycérol ou éthyl diglycol.

Le document EP-A-806 202 décrit l'utilisation d'un agent stabilisant du type hydroxypropylméthyl cellulose dans une matrice lipophile, dont la cinétique de libération du principe actif est, du fait de l'érosion de ladite matrice, plus ou moins régulière. Ce document ne concerne donc pas une composition susceptible de créer une émulsion et encore moins une micro-émulsion mais une matrice lipidique libérant le principe actif par érosion.

Le document US-A-5 206 219 décrit une composition pharmaceutique à base de principe actif susceptible de former une microémulsion in situ dans le tractus intestinal. La composition décrite comprend, en tant que phase liphile, des phospholipides et non pas des glycérides polyglycosylés et des agents tensio-actifs à HLB élevée, voisine de 18, conduisant à la formation de véritables émulsions et non de microémulsions.

En d'autres termes, le problème que se propose de résoudre l'invention est de fournir une composition du type SMEDDS® apte à libérer progressivement le ou les actifs qu'elle véhicule, et ce quelle que soit la consistance du SMEDDS® à température ambiante, solide ou liquide.

Pour résoudre ce problème, l'invention propose une composition à libération prolongée du principe actif à usage pharmaceutique ou cosmétique destinée à être ingérée comprenant un système auto-micro-émulsionable au contact d'une phase hydrophile apportée, après ingestion, par le liquide physiologique, ledit système auto-micro-émulsionable comprenant :
- au moins un actif,
- une phase lipophile constituée d'un mélange de mono-, di- et triglycérides et des acides gras en C₈-C₁₈ et de mono- et diesters de polyéthylène glycol présentant une balance hydrophile/lipophile (HLB) inférieure à 16;
- un agent tensio-actif (TA) à base de glycérides présentant une HLB inférieure à 16 choisi dans le groupe comprenant les glycérides polyglycolysés saturés en C₈-C₁₀ et les esters oléïques du polyglycérol ;
- un agent cotensio-actif (CoTA) choisi dans le groupe comprenant les esters d'acides gras du propylène glycol, les esters oléïques du polyglycérol, l'éthyl diglycol ;
- le rapport TA-CoTA étant compris entre 0,5 et 6.

La composition de l'invention se caractérise en ce qu'elle comprend en outre une matrice polymérique inerte non ionisable à pH physiologique, dispersée dans le système auto-micro-émulsionable avant ingestion, ladite matrice polymérique étant apte, après ingestion, à former, au contact du liquide physiologique, une matrice polymérique gélifiée permettant de libérer par diffusion,de façon continue et prolongée, l'actif alors micro-émulsionné.

Dans la suite de la description et dans les revendications, par "actif micro-émulsionné", on désigne l'actif solubilisé au coeur de la micro-émulsion, c'est-à-dire dans sa zone hydrophobe.

De même, par l'expression « liquide physiologique», on désigne le milieu physiologique in vivo, tel qu'il se présente après ingestion de la composition et dont le pH variera en fonction de l'état du tractus gastro-intestinal.

Cependant, au stade expérimental, c'est-à-dire sans ingestion de la composition, le liquide physiologique est remplacé par de l'eau ou un milieu physiologique in vitro reconstitué. Dans ce cas, la micro-émulsion se formera au simple contact de la phase aqueuse.

Dans la suite de la description et dans les revendications, par l'expression "glycérides polyglycolysés", on désigne un mélange de mono-, di- et triglycérides et de mono- et diesters de polyéthylène glycol (PEG) de poids moléculaire compris de préférence entre 200 et 600, éventuellement de glycérol et de PEG libre, dont on règle la valeur HLB par longueur de la chaîne du PEG et dont on règle le point de fusion par la longueur des chaînes des acides gras, du PEG et des degrés de saturation des chaînes grasses, donc de l'huile de départ.

De même, par l'expression "acides gras en C₈ à C₁₈", que l'on écrit aussi C₈-C₁₈, on désigne des mélanges en proportions significatives et variables des acides capryliques (C₈), capriques (C₁₀), lauriques (C₁₂), myristiques (C₁₄), palmitiques (C₁₆) et stéariques (C₁₈), lorsque ces acides sont saturés et les acides insaturés correspondants en C₈-C₁₈.

Il est rappelé que les proportions de ces acides gras peuvent varier en fonction des huiles de départ.

Dans une forme préférée de réalisation de l'invention, l'agent cotensio actif est choisi dans le groupe comprenant les esters lauriques du propylène glycol, les esters capriques du propylène glycol et les esters palmitiques du propylène glycol.

En d'autres termes, l'invention consiste à incorporer une matrice polymérique inerte au sein d'un système auto-micro-émulsionable. Ledit système auto-micro-émulsionable forme alors une micro-émulsion après ingestion, au contact du liquide physiologique, permettant ainsi par diffusion à travers la matrice alors gélifiée au contact du liquide physiologique de libérer progressivement de façon continue et régulière le ou les actifs micro-émulsionnés.

En pratique, lorsque la composition de l'invention est formulée en gélules, après ingestion, la gélule se dissout au contact des fluides digestifs, entraînant parallèlement la formation d'une petite fraction de micro-émulsion, puisque l'eau entre progressivement en contact avec le SMEDDS®. Il s'ensuit qu'une faible partie du principe actif est alors microémulsionnée, puis le principe actif microémulsionné libéré.

Concomitamment, le polymère va progressivement s'hydrater, puis se gélifier, entraînant ainsi la formation d'une couche visqueuse, dont le volume va progressivement augmenter pour former une matrice à partir de laquelle la micro-émulsion est libérée par diffusion. La barrière gélifiée ainsi constituée s'oppose donc à la libération rapide du principe actif micro-émulsionné et en contrôlant la pénétration de l'eau de l'extérieur vers l'intérieur, permet de libérer progressivement ledit principe actif micro-émulsionné.

Par ailleurs, dans le cas où le SMEDDS® comprend des corps gras de HLB > 10, on a constaté que de façon tout-à-fait surprenante la matrice polymérique est gelifiée seulement après ingestion, c'est-à-dire au contact du liquide physiologique, alors qu'on aurait pu s'attendre à ce que la gélification intervienne avant l'ingestion au seul contact des constituants du SMEDDS®, dont la valeur HLB très élevée (> 10) avait pu entraîner la solvatation du polymère, et ainsi conduire à la formation d'un gel.

De plus, il n'était pas évident que le taux de libération d'actif ne fluctue pas en fonction des conditions hydrodynamiques, c'est-à-dire en fonction de la motilité intestinale, notamment par érosion de la matrice, comme cela se produit pour les systèmes monolithiques. Au contraire, la composition selon l'invention présente l'avantage d'être indépendante des conditions hydrodynamiques, puisque la matrice au contact du liquide physiologique gonfle et forme un réseau continu, permettant de libérer les micelles constituant la micro-émulsion.

Dans une première forme de réalisation de l'invention, pour que la matrice polymérique forme un réseau hydraté et donc un gel au contact du liquide physiologique, la matrice polymérique est choisie dans le groupe comprenant les polymères cellulosiques.

Selon cette forme de réalisation, la matrice polymérique a un poids moléculaire inférieur à un million.

Pour un poids moléculaire supérieur à un million, la viscosité de la composition est trop élevée et la libération du SMEDDS® est améliorée

Avantageusement, le poids moléculaire de la matrice polymérique est compris entre 80 000 et 800 000.

Dans une seconde forme de réalisation de l'invention, la matrice polymérique est choisie dans le groupe comprenant les polymères acryliques. Tous les polymères acryliques susceptibles de gonfler au contact d'une phase aqueuse pourront convenir.

Dans une troisième forme de réalisation de l'invention, la matrice polymérique est choisie dans le groupe comprenant les polysaccharides non cellulosiques, par exemple les gommes.

Lorsque l'on désire incorporer dans le SMEDDS® un actif hydrophobe, on met en oeuvre une matrice polymérique hydrophile choisie dans le groupe comprenant l'hydroxypropyl cellulose, l'hydroxypropylméthyl cellulose, la méthylcellulose seuls ou en mélange.

De même, lorsque l'on désire incorporer un actif hydrophile dans le SMEDDS®, on met en oeuvre une matrice polymérique hydrophobe du type éthyl cellulose.

La composition de l'invention permet ainsi de mettre en oeuvre des actifs hydrophiles indépendamment de leur hydrosolubilité, puisque la libération de l'actif n'est pas réalisé en fonction de son hydrosolubilité, mais par diffusion de micelles, l'actif étant préalablement présenté au sein d'une forme de SMEDDS®.

Pour provoquer la gélification de la matrice polymérique au contact du liquide physiologique, tout en assurant la formation de la micro-émulsion, la matrice polymérique inerte représente de 0,5 à 40 % en poids de la composition totale.

Pour une concentration inférieure à 0,5 %, on n'observe pas de gélification de la matrice au contact du liquide physiologique. Au contraire, pour une concentration en matrice supérieure à 40 %, la solution devient trop visqueuse.

Avantageusement, la concentration en matrice polymérique est comprise entre 5 et 25 % en poids de la composition totale.

L'invention se rapporte également au procédé pour la fabrication de la composition.

Selon ce procédé :
- on prépare tout d'abord un système auto-micro-émulsionable en mélangeant, le cas échéant tout en chauffant, sous agitation, l'actif, la phase lipophile, l'agent tensio-actif et l'agent cotensio-actif ;
- puis, toujours sous agitation, on disperse progressivement la matrice polymérique sous forme de poudre au sein dudit système auto-micro-émulsionable.

La composition de l'invention, qui à température ambiante, est à l'état liquide ou semi-solide en fonction de la nature des constituants du SMEDDS®, sera formulée en gélule.

Dans ce cas, celles-ci seront remplies de la composition de l'invention à l'état liquide, éventuellement préalablement chauffée, laquelle lors du refroidissement et en fonction de la composition du SMEDDS® pourra se solidifier à température ambiante.

L'invention concerne donc également la gélule incorporant le SMEDDS® à libération prolongée décrit ci-avant.

L'invention et les avantages qui en découlent ressortiront mieux de l'exemple de réalisation suivant à l'appui des figures annexées.

La figure 1 est une représentation schématique du système de libération de l'actif à partir de la composition de l'invention.

La figure 2 est une représentation des courbes de dissolution d'indometacine à pH 1,2 pour l'indometacine seule, l'indometacine formulée sous forme de SMEDDS® tel que décrit dans le document EP-A-0 670 715 et l'indometacine formulée sous forme de SMEDDS® à libération prolongée tel que décrit dans la présente invention.

### Exemple 1

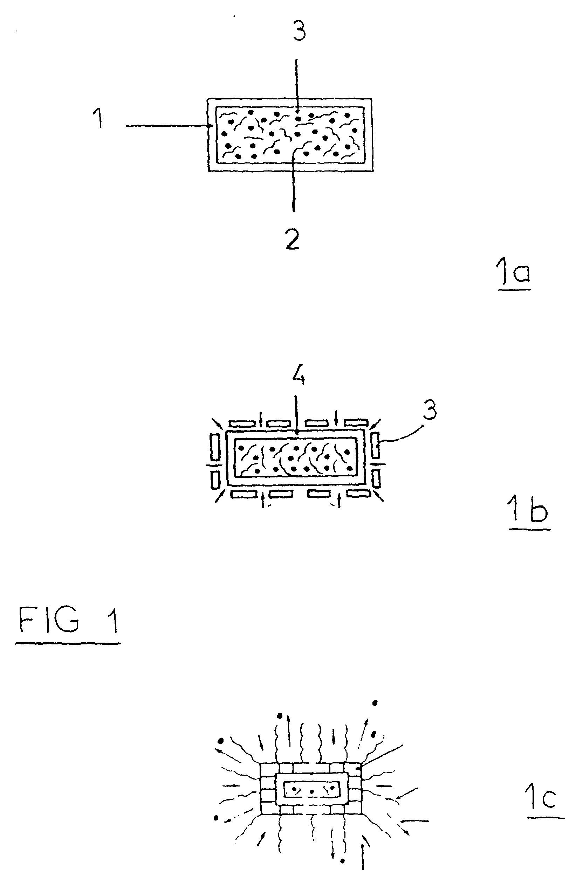

Comme déjà dit, la figure 1 montre le fonctionnement de la matrice polymérique dispersée dans une formule de type SMEDDS®, la composition obtenue se présentant sous forme de gélule.

Sur la figure 1a, on a représenté une gélule (1) renfermant une matrice polymérique (2) répartie de façon homogène et à l'état sec dans la masse et un système auto-micro émulsionnable (3) ou SMEDDS®.

Comme le montre la figure 1b, au contact de la phase hydrophile, c'est-à-dire du liquide physiologique, la gélule (1) est dissoute, ce qui permet au liquide physiologique de s'introduire au sein du mélange de polymère et de SMEDDS®.

Au contact de l'eau, la matrice polymérique forme progressivement en se structurant une barrière gélifiée (4).

Comme le montre la figure 1c, la matrice polymérique se déstructure progressivement permettant ainsi de libérer lentement le SMEDDS®.

### Exemple 2

L'essai suivant a été réalisé en laboratoire en mettant en oeuvre l'eau comme milieu physiologique.

### - Fabrication de la composition de l'invention sous forme de gélule

Dans cet exemple, on prépare un SMEDDS® dont la composition est la suivante :

| | | |
|---|---|---|
| tensioactif | LABRASOL | 43,40 % |
| cotensioactif | PLUROLOLEIQUE | 14,40 % |
| phase lipophile | LABRAFIL WL2609BS | 38,40 % |
| principe actif | INDOMETACINE | 4 %. |

De façon connue, les constituants du SMEDDS® sont mélangés à température ambiante sous agitation comprise entre 60 et 100 tours par minute.

On disperse ensuite progressivement toujours sous agitation la matrice polymérique constituée d'Hydroxypropylméthylcellulose (HPMC) représentant 20% de la composition finale.

On obtient une préparation liquide qui est ensuite formulée en gélules.

### - Courbes de dissolution

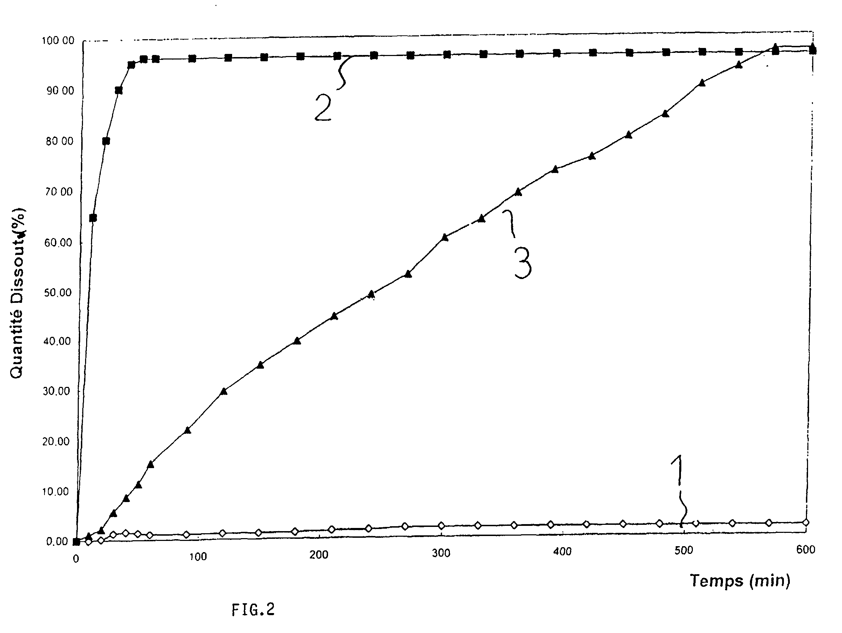

Sur la figure 2, on a représenté les courbes de dissolution de l'indometacine lorsque l'actif est seul (courbe 1), se présente sous forme de SMEDDS® à libération immédiate (courbe 2), ou se présente sous forme de SMEDDS® à libération prolongée selon l'invention (courbe 3).

Comme le montre cette figure, l'indometacine dont on sait qu'elle est insoluble à pH acide (courbe 3), peut être rendue parfaitement disponible lorsqu'elle est incorporée dans un SMEDDS® (courbe 1 et 2).

Comme le montre la courbe 2, la composition de l'invention présente des caractéristiques de libération prolongée et ce malgré le fait que le SMEDDS® se présente à l'état liquide à température ambiante.

### - Caractérisation de la micro-émulsion formée par la composition de l'invention

### Effet TYNDALL

La microémulsion obtenue in vitro présente un aspect bleuté qui correspond à l'effet TYNDALL.

### Taille des gouttelettes formant la micro-émulsion

On a mesuré par spectroscopie à corrélation de photons la taille des gouttelettes de la micro-émulsion obtenue in vitro lorsque la composition de l'invention est mise au contact d'une phase aqueuse.

La taille moyenne des gouttelettes obtenue est de 25 nanomètres.

Les avantages de l'invention ressortent bien de la description ci-avant.

On notera en particulier la possibilité d'incorporer des SMEDDS® au sein de formes à libération prolongée permettant d'augmenter la biodisponibilité de l'actif.

On note également que de façon tout à fait surprenante, la composition de l'invention présente une structure telle qu'elle permet de libérer progressivement le principe actif in vivo et ce même si le SMEDDS® se présente sous forme de liquide à température ambiante.

## Revendications

1. Composition destinée à être ingérée comprenant un système auto-micro-émulsionable au contact d'une phase hydrophile apportée, après ingestion, par le liquide physiologique, ledit système auto-micro-émulsionable comprenant :
• au moins un actif ;
• une phase lipophile constituée d'un mélange de mono-, di- et triglycérides avec des acides gras en C₈-C₁₈ et de mono- et diesters de polyéthylène glycol présentant une balance hydrophile/lipophile (HLB) inférieure à 16;
• un agent tensio-actif (TA) à base de glycérides présentant une HLB inférieure à 16 choisi dans le groupe comprenant les glycérides polyglycolysés saturés en C₈-C₁₀ et les esters oléïques du polyglycérol ;
• un agent cotensio-actif (CoTA) choisi dans le groupe comprenant les esters d'acides gras du propylène glycol, les esters oléïques du polyglycérol, et l'éthyl diglycol ;
• le rapport TA-CoTA étant compris entre 0,5 et 6 ;
***caractérisée* en ce que** ladite composition comprend en outre une matrice polymérique inerte, représentant de 0,5 à 40 % en poids de la composition totale, non ionisable à pH physiologique, dispersée dans le système auto-micro-émulsionable avant ingestion, ladite matrice polymérique étant apte, après ingestion, à former, au contact du liquide physiologique, une matrice polymérique gélifiée permettant de libérer par diffusion, de façon continue et prolongée l'actif alors micro-émulsionné.

2. Composition selon la revendication 1, **caractérisée ce que** l'agent cotensio-actif est choisi dans le groupe comprenant les esters lauriques du propylène glycol, les esters capriques du propylène glycol et les esters palmitiques du propylène glycol.

3. Composition selon l'une des revendications précédentes, **caractérisée ce que** la matrice polymérique est choisie dans le groupe comprenant les polymères cellulosiques.

4. Composition selon la revendication 3, **caractérisée en ce que** le poids moléculaire de la matrice polymérique est inférieur à un million.

5. Composition selon la revendication 4, **caractérisée en ce que** le poids moléculaire de la matrice polymérique est compris entre 80 000 et 800 000.

6. Composition selon l'une des revendications 2 à 5, **caractérisée en ce que** la matrice polymérique est hydrophile et choisie dans le groupe comprenant l'hydroxypropyl cellulose, l'hydroxypropylméthyl cellulose, le méthyl cellulose seuls ou en mélange.

7. Composition selon l'une des revendications 2 à 5, **caractérisée en ce que** la matrice polymérique est hydrophobe et constituée d'éthyl cellulose.

8. Composition selon l'une des revendications 1 ou 2, **caractérisée en ce que** la matrice polymérique est choisie dans le groupe comprenant les polymères acryliques susceptibles de gonfler au contact d'une phase aqueuse.

9. Composition selon l'une des revendications 1 ou 2, **caractérisée en ce que** la matrice polymérique est choisie dans le groupe comprenant les polysaccharides non cellulosiques.

10. Composition selon la revendication 9, **caractérisée en ce que** la matrice polymérique inerte représente de 5 à 25 % en poids de la composition totale.

11. Procédé pour la fabrication de la composition objet des revendications 1 à 10, dans lequel :
• on prépare tout d'abord un système auto-micro-émulsionable en mélangeant, le cas échéant tout en chauffant, sous agitation, l'actif, une phase lipophile, un agent tensio-actif et un agent cotensio-actif ;
• puis, toujours sous agitation, on disperse progressivement la matrice polymérique sous forme de poudre au sein dudit système auto-micro-émulsionable.

12. Gélule incorporant la composition objet de l'une des revendications 1 à 10.

## Claims

1. Composition intended to be ingested, comprising a system which is self-microemulsifying on contact with a hydrophilic phase provided, after ingestion, by the physiological fluid, the said self-microemulsifying system comprising:
• at least one active agent,
• a lipophilic phase consisting of a mixture of mono-, di- and triglycerides and of C₈-C₁₈ fatty acids and of polyethylene glycol monoesters and diesters with a hydrophilic/lipophilic balance (HLB) of less than 16;
• a glyceride-based surfactant (SA) with an HLB of less than 16, chosen from the group comprising saturated C₈-C₁₀ polyglycosylated glycerides and oleic esters of polyglycerol;
• a co-surfactant (CoSA) chosen from the group comprising fatty acid esters of propylene glycol, oleic esters of polyglycerol, and ethyl diglycol;
• the SA-CoSA ratio being between 0.5 and 6;
**characterized in that** the said composition also comprises an inert polymer matrix, representing from 0.5% to 40% relative to the weight of the total composition, which is not ionizable at physiological pH, dispersed in the self-microemulsifying system before ingestion, the said polymer matrix being capable, after ingestion, of forming, in contact with physiological fluid, a gelled polymer matrix making it possible to release the thus microemulsified active agent in a continuous and sustained manner by diffusion.

2. Composition according to Claim 1, **characterized in that** the co-surfactant is chosen from the group comprising lauric esters of propylene glycol, capric esters of propylene glycol and palmitic esters of propylene glycol.

3. Composition according to either of the preceding claims, **characterized in that** the polymer matrix is chosen from the group comprising cellulose polymers.

4. Composition according to Claim 3, **characterized in that** the molecular weight of the polymer matrix is less than one million.

5. Composition according to Claim 4, **characterized in that** the molecular weight of the polymer matrix is between 80,000 and 800,000.

6. Composition according to one of Claims 2 to 5, **characterized in that** the polymer matrix is hydrophilic and chosen from the group comprising hydroxypropylcellulose, hydroxypropylmethylcellulose and methylcellulose, alone or as a mixture.

7. Composition according to one of Claims 2 to 5, **characterized in that** the polymer matrix is hydrophobic and consists of ethylcellulose.

8. Composition according to either of Claims 1 and 2, **characterized in that** the polymer matrix is chosen from the group comprising acrylic polymers which are capable of swelling on contact with an aqueous phase.

9. Composition according to either of Claims 1 and 2, **characterized in that** the polymer matrix is chosen from the group comprising non-cellulose polysaccharides.

10. Composition according to Claim 9, **characterized in that** the inert polymer matrix represents from 5% to 25% relative to the weight of the total composition.

11. Process for manufacturing the composition which is the subject of Claims 1 to 10, in which:
• a self-microemulsifying system is first prepared by mixing, if necessary while heating, with stirring, the active agent, a lipophilic phase, a surfactant and a co-surfactant;
• the polymer matrix in powder form is then gradually dispersed, while still stirring, in the said self-microemulsifying system.

12. Gel capsule incorporating the composition which is the subject of one of Claims 1 to 10.

## Patentansprüche

1. Einzunehmende Zusammensetzung mit einem System, das bei Kontakt mit einer nach Einnahme durch die physiologische Flüssigkeit gelieferten hydrophilen Phase selbstmikroemulgierbar ist, wobei das genannte selbstmikroemulgierbare System umfasst:
• wenigstens einen Wirkstoff;
• eine lipophile Phase bestehend aus einer Mischung aus Mono-, Di- und Triglyceriden mit C₈-C₁₈-Fettsäuren und aus Polyethylenglykolmono- und - diestern, die eine hydrophil-lipophile Balance (HLB) kleiner 16 aufweisen;
• ein Tensid (TA) auf der Basis von Glyceriden mit einer HLB kleiner 16, das ausgewählt ist aus der Gruppe enthaltend gesättigte, polyglykolisierte C₈-C₁₀-Glyceride und Oleinester des Polyglyzerin;
• ein Co-Tensid (CoTA) ausgewählt aus der Gruppe enthaltend Fettsäureester des Propylenglykol, Oleinester des Polyglyzerin und Ethyldiglykol;
• wobei das Verhältnis von TA zu CoTA zwischen 0,5 und 6 liegt;
**dadurch gekennzeichnet, dass** die genannte Zusammensetzung außerdem eine bei physiologischem pH-Wert nicht ionisierbare, inerte Polymermatrix enthält, die 0,5 bis 40 Gew.% der Gesamtzusammensetzung ausmacht und vor Einnahme in dem selbstmikroemulgierbaren System dispergiert ist, wobei die genannte Polymermatrix nach Einnahme in der Lage ist, bei Kontakt mit der physiologischen Flüssigkeit eine gelierte Polymermatrix zu bilden, die es ermöglicht, den nun mikroemulgierten Wirkstoff durch Diffusion stetig und verlängert freizusetzen.

2. Zusammensetzung nach Anspruch 1, **dadurch gekennzeichnet, dass** das Co-Tensid ausgewählt ist aus der Gruppe enthaltend die Laurinester des Propylenglykols, die Caprinester des Propylenglykols und die Palmitinester des Propylenglykols.

3. Zusammensetzung nach einem der vorhergehenden Ansprüche, **dadurch gekennzeichnet, dass** die Polymermatrix ausgewählt ist aus der Zellulosepolymere enthaltenden Gruppe.

4. Zusammensetzung nach Anspruch 3, **dadurch gekennzeichnet, dass** das Molekulargewicht der Polymermatrix unter einer Million liegt.

5. Zusammensetzung nach Anspruch 4, **dadurch gekennzeichnet, dass** das Molekulargewicht der Polymermatrix zwischen 80.000 und 800.000 liegt.

6. Zusammensetzung nach einem der Ansprüche 2 bis 5, **dadurch gekennzeichnet, dass** die Polymermatrix hydrophil und ausgewählt ist aus der Gruppe enthaltend Hydroxypropylzellulose, Hydroxypropylmethylzellulose, Methylzellulose einzeln oder als Mischung.

7. Zusammensetzung nach einem der Ansprüche 2 bis 5, **dadurch gekennzeichnet, dass** die Polymermatrix hydrophob und von Ethylzellulose gebildet ist.

8. Zusammensetzung nach einem der Ansprüche 1 oder 2, **dadurch gekennzeichnet, dass** die Polymermatrix ausgewählt ist aus der Gruppe enthaltend Acrylpolymere, die sich bei Kontakt mit einer wässrigen Phase aufblähen können.

9. Zusammensetzung nach einem der Ansprüche 1 oder 2, **dadurch gekennzeichnet, dass** die Polymermatrix ausgewählt ist aus der Gruppe enthaltend nicht zellulosehaltige Polysaccharide.

10. Zusammensetzung nach Anspruch 9, **dadurch gekennzeichnet, dass** die inerte Polymermatrix zwischen 5 und 25 Gew. % der Gesamtzusammensetzung ausmacht.

11. Verfahren zur Herstellung der Zusammensetzung nach den Ansprüchen 1 bis 10, bei dem:
• zunächst ein selbstmikroemulgierbares System hergestellt wird, indem der Wirkstoff, eine lipophile Phase, ein Tensid und ein Co-Tensid unter Rühren, ggf. unter gleichzeitigem Erhitzen, gemischt werden;
• anschließend unter weiterem Rühren nach und nach die Polymermatrix in Pulverform innerhalb des genannten selbstmikroemulgierbaren Systems dispergiert wird.

12. Kapsel, welche die Zusammensetzung nach einem der Ansprüche 1 bis 10 aufnimmt.
